# EUROPEAN PATENT APPLICATION

(11) **EP 3 326 531 A1**
(43) Date of publication of application: **30.05.2018**
(21) Application number: 16200651.4
(22) Date of filing: 25.11.2016
(51) Int. Cl.: A61B 5/22, A61B 5/11, A61N 1/36

(54) **MEDICAL DEVICE, KIT AND METHOD FOR SENSING A THUMB COMPRESSION FORCE**

(71) Applicant: Haute École Du Paysage D' Ingénierie Et D' Architecture (Hepia), 1202 Genève (CH)
(72) Inventor: PASSERAUB, Philippe Alfons, 1680 Romont (CH); MOREILLON, Fabien, 1227 Carouge (CH); D'HOLLANDER, Alain, 74160 Saint-Julien-en-Genevois (FR)
(74) Representative: KATZAROV S.A.

(57) **Abstract**

Compression sensing device (1) for measuring an isometric thumb compression force applied by a thumb of a conscious or unconscious patient comprising a handle (3) to be gripped by a hand of the patient, a force sensor (31), and a thumb support (2) mounted at a first end of the handle, for receiving a thumb of the hand of the patient in zero preload conditions and adapted to transmit compression force of the thumb to the force sensor, characterized in that it further comprises a protective shield (4) attached to the handle and at least partially surrounding the thumb support so as to protect it from any external artefactual compression.

## Description

### Technical field

The invention relates to a medical device for sensing a thumb compression force, a set comprising said medical device and a method using the same. More particularly, the invention concerns a medical device for sensing an isometric thumb compression force in zero preload conditions. The device can be used for monitoring the evolution of a patient's condition during a general anaesthesia using neuro-muscular relaxants and in any clinical situation needing quantitative information concerning voluntary maximal or residual forces.

### Background Art

It is very well known that, in case of surgery and more particularly in case of heavy surgery, general anaesthesia is mandatory. In many cases, the use of a curare is indicated in order to generate a controlled and selective hypotonic state of the voluntary or striated muscles thereby facilitating the care process safety. The action of this conventional drug consists in blocking, overall at the muscle level, the action of the chemical signals issued from the motor nerves. For safety reasons, the effects of the curare must be precisely controlled during the whole surgery and to assure, thereafter, an awakening period free of residual paralysis. In order to achieve this, the patient's condition must be monitored also during the awakening.

Several techniques can be used to monitor the occurrence of residual paralysis. These techniques consist in the measure of a thumb distal phalange acceleration upon electrical stimulation, a kynemyography process which consists in measuring the acceleration of the thumb against a resistive material, a surface electromyography or measuring a force of compression of the thumb with or without pre-constraint. However these techniques are limited and not completely reliable because their repeatability is not optimum as artefacts remain too numerous.

Moreover during the awakening period, the residual paralysis is assessed, nowadays, only through low frequency (2 Hz) electric stimulations generating a poorly sensitive parameter for detecting very low level of residual paralysis. The use of this technique is not satisfying because it is not optimum from a patient security point of view. There is therefore a need for a reliable monitoring of the patient during awakening.

For example, document EP0283387 describes a thumb pressure measurement device comprising a handle and a thumb support where the thumb support is placed perpendicular to the handle's longitudinal axis so as to monitor a pressure due to an adduction movement of the thumb which is not an isometric measure. Further, the device must use software to correct parasitic measurements due to external factors. Finally, the arm is attached through 2 bindings, but the hand is not reliably attached to the device.

Conventional devices and methods generally use electrical stimulations that can be divided into two groups. One group is using exclusively the non-tetanic frequency stimulations having a frequency generally lower than 10 Hz while the other one considers also real tetanic frequency stimulations having a frequency higher than 30 Hz. A complete neuro-muscular blockade monitoring would be able to give complete information to the clinicians; this includes the display of low and high frequencies evoked signals.

It is therefore an object of the invention to provide a medical device for sensing a thumb compression force overcoming the above defects and more particularly, to provide a medical device for sensing a thumb compression force having a great modularity, adaptable to any hand size or shape, with no parasitic noise/pressures, and providing reliable isometric measurement of the thumb compression measured either in a conscious person or in an anesthetized patient using low and high frequencies stimulations.

### Object of the Invention

This and further objects are achieved by the present invention described below.

Accordingly, a first aspect of the present invention relates to a compression sensing device for measuring an isometric thumb compression force applied by a thumb of a conscious or unconscious patient comprising a handle to be gripped by a hand of the patient, a force sensor, and a thumb support mounted at a first end of the handle, for receiving a thumb of the hand of the patient in zero preload conditions and adapted to transmit the compression force of the thumb to the force sensor, characterized in that it further comprises a protective shield attached to the handle and at least partially surrounding the thumb support so as to protect it from any external artefactual compressions. This compression device can therefore be used in any situation while the measurements are not subjected to parasitic values as the thumb and the thumb support are protected by a protective shield.

According to a preferred implementation of the present invention, the compression sensing device further comprises a hand support movably mounted on a second end of the handle opposed to the first end and being sloped with respect to said handle so as to move along and around the handle's longitudinal direction. The stable positioning of the hand is therefore assured and the device can be adapted to any size of hands.

Preferably, the thumb support is a bored component disposed so as to be rotatable around the longitudinal axis of the handle. Thanks to this, one can enhance modularity of the device being adapted for both hands.

In a particularly preferred solution, the bored component is a cylindrical element having a sloped support surface adapted to receive the two phalanges of the thumb. This particular configuration permits to have the thumb in a particular position maximizing its compression force, and to provide an element keeping the thumb in place during monitoring.

According to a preferred embodiment of the present invention, the zero preload positioning of the thumb on its support permits to transmit integrally the isometric compression force of the thumb to the force sensor, which has a limited compression run. With this configuration, the measurements have an excellent repeatability.

Advantageously, the protective shield comprises an opening above the thumb support, adapted for receiving a calibration tool for calibration. It is therefore possible to calibrate or re-calibrate the compression sensing device even with a hand in place.

Preferably, the protective shield is detachably attached to the handle. Thanks to this, one can enhance modularity of the device.

In a particularly preferred embodiment, the compression sensing device further comprises binding means adapted to maintain the hand in position on the compression sensing device. This permits to lower the risk of having false results.

According to a preferred embodiment of the present invention, the binding means are attached on the protective shield on one end and on the hand support on the other end. Therefore, it can be adapted to the different positions of the hand support to fit any type of hand.

Advantageously, the binding means comprise at least two non-elastic tapes disposed so as to cross each other at the metacarpophalangeal joint of the patient's hand upon use. With this specific arrangement, the thumb of the patient is particularly well maintained in place. This lowers the risks of having the thumb releasing the compression sensing device.

According to a preferred embodiment of the present invention, the force sensor is provided inside the handle. Such a configuration results in less miniaturization requirements on the force sensor.

A second aspect of the present invention relates to a compression sensing set comprising the compression sensing device according to the first aspect of the present invention, a processing module adapted to process the compression detected by the compression sensing device, and a display module adapted to display the signals recorded and the calculated results. The advantages of the compression sensing set are similar to the ones of the compression sensing device of the first aspect of the present invention and will not be repeated here.

Preferably, the compression sensing set further comprises electrical stimulation generating means. Therefore, one can monitor, on the thumb, the patient's residual paralysis according various electrical stimulations.

According to a preferred embodiment of the present invention, the electrical generating means are adapted to generate several different stimulation profiles having a frequency lower than 10 Hz and/or larger than 30 Hz. Thanks to this, one can generate several types of signals and parameters for an improved monitoring process, particularly useful for the detection of low level of residual paralysis.

A third aspect of the present invention relates to a compression sensing method using the compression sensing kit according to the second aspect of the present invention comprising the steps of detecting a compression force applied by the thumb on the force sensor through the thumb support, converting the detected compression into a standard force, converting said standard force into an electrical signal and sending it to the processing means, and displaying the detected force. The advantages of the compression sensing method are similar to the ones of the compression sensing device of the first aspect of the present invention and will not be repeated here.

### Description of the Drawings

The invention will now be described in relation to the accompanying figures that are illustrative only and should not in any case be considered as limitative, wherein:
Figure 1 represents a compact view of the compression sensing device according to a preferred embodiment of the invention.
Figure 2 illustrates an exploded view of the compression sensing device according to a preferred embodiment of the invention.

### Detailed Description of the Preferred Embodiments of the Present Invention

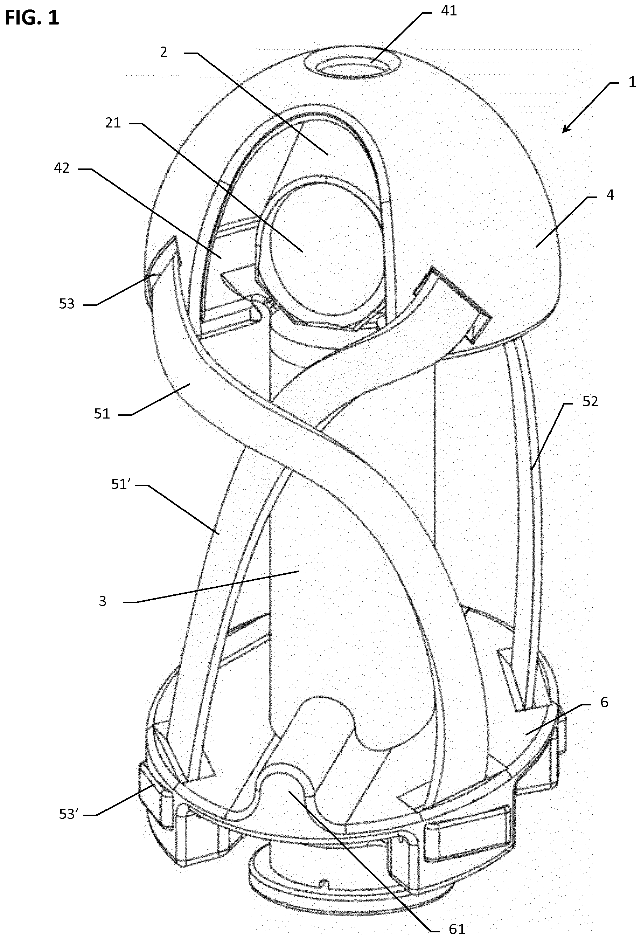

Figure 1 illustrates a compact view of the compression sensing device 1 for sensing an isometric thumb compression force applied by a thumb of a patient (not represented). Preferably, the thumb compression force is caused by an electrical stimulation at the patient's hand which in turn generates a contraction of muscles causing the thumb compression, but the invention is not limited thereto and the compression force can be an intended compression actively generated by a conscious patient.

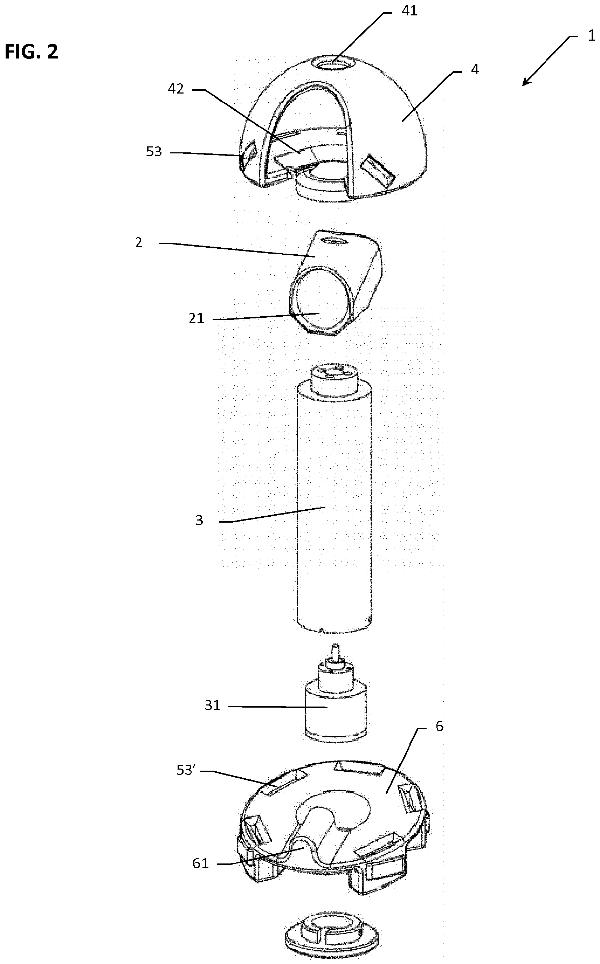

The compression sensing device 1 of this preferred embodiment comprises a handle 3 to be gripped by a hand of the patient, a force sensor (not represented in figure 1 and 31 in figure 2) preferably provided inside the handle 3, but it can be provided elsewhere, for example within the thumb support, and a thumb support 2 mounted at a first end of the handle 3, for receiving a thumb of the hand of the patient in zero preload conditions and adapted to transmit compression force of the thumb to the force sensor 31. Preferably, the thumb support 2 is a bored component having an inner cylindrical shape and having a sloped support surface 21 adapted to receive the two phalanges of the thumb, it is preferably disposed so as to be rotatable around the longitudinal axis of the handle 3. Although this is not inherently represented in the figures, the thumb support 2 is preferably adapted to have a limited compression run so as to transmit an isometric compression force of the thumb to the force sensor 31, and the zero preload positioning of the thumb on its support 2 permits to integrally transmit the isometric compression force of the thumb to the force sensor 31 which has a limited compression run. This ensures having measurements with a good repeatability. One must understand here that the term "limited compression run" should be understood as the distance along which the thumb moves upon compression that is limited to a few microns to have a good measurement repeatability in case of these quasi-isometric contractions.

A further important aspect here is the comfort provided by the sensing device to the patient when he holds the sensing device. One of the key features regarding the comfort is the position relation between the thumb support and the handle. More particularly, in order to provide a particular holding comfort, the thumb support is sloped with respect to the handle and presents a smooth rounded angled shape at its lower edge thereby preventing the injury risk due for example by pinching between the thumb support and the handle or the like.

The slope of the thumb support with respect to the handle is preferably comprised in a range between 0° and 90°, preferably from 5° to 80°, more preferably from 5° to 60°, more preferably from 10° to 40°, even preferably from 15° to 30°, still more preferably from 20° to 25°, most preferably the slope is 25°. The slope is these ranges more and more corresponds to a preferred position of the muscles of the thumb so as to maximize the compression force thereby improving the obtained results.

Furthermore, the compression sensing device 1 further comprises a protective shield 4 attached to the handle 3 and at least partially surrounding the thumb support 2 so as to protect it from any external artefactual compression. The term "surrounding" here should not be understood as laterally situated but should more generally be understood as being located, at any suitable distance, around and/or above and/or under the thumb support 2. Also, the term "surrounding" should not be understood as completely surrounding because a protective shield 4 completely surrounding the thumb support 2 would impede the insertion of said thumb into the thumb support 2 but should be understood as surrounding so as to sufficiently protect the thumb support according to the situation. For example, it can surround the thumb support by having some kind of mushroom shape or hemispherical shape (as represented). One could even provide a protective shield 4 having high lateral walls without "roof" if this permits to impede parasitic measurements. Here the expression "external artefactual compression" obviously does not refer to the thumb compression but to any other measurement that are not proper to monitor an awakening for example and more generally to any compression that are not desired so that the patient can grip the device and then fall asleep and measurements are not altered during the sleep by any erratic movements.

This compression device 1 can therefore be used in any situation while the measurements are not subjected to parasitic values since the thumb support 2 and the thumb are protected by a protective shield 4. The protective shield preferably comprises an opening 41 above the thumb support 2, adapted for receiving a calibration tool (not represented) for calibration, it is therefore possible to calibrate the compression sensing device 1 without dismantling it. The protective shield 4 can optionally be detachably attached to the handle 3. Finally, it has to be noted that the illustrated shield 4 has a hemispherical shape which has the advantage of not providing corners and therefore being safe. However, the invention is clearly not limited to this shape. Preferably, the protective shield 4 is attached to the handle through a flat element 42 also protecting the thumb support and providing a pivotable behaviour about the longitudinal axis of the handle to the protective shield 4. This pivotable behaviour can be provided through a specific mechanism between the protective shield and the element 42, in which case the element 42 is not pivotable, but if needed the element 42 can be fixedly attached to the protective shield so as to move with it and a pivotable mechanism can be provided between the handle 3 and the element 42. Finally, it comprises attachments 53 for bindings that will be described later.

Also, the compression sensing device 1 preferably further comprises a hand support 6 mounted on a second end of the handle 3 opposed to the first end and being sloped with respect to said handle 3 and movable along and around the handle's longitudinal direction so as to adapt to any size of hands. The stable positioning of the hand is therefore assured.

This hand support 6 also comprises a protuberance 61 protruding toward the protective shield 4. This protuberance locks passively the cubital side of any left or right palm to assure a stable positioning of the hands.

In figure 1 one can see that the device further comprises binding means 51, 51', 52 adapted to maintain the hand in position on the compression sensing device 1. Preferably, the binding means are attached on the protective shield 4 on one end through the fixing means 53 and on the hand support 6 on the other end through fixing means 53' that can release the binding means 51, 51', 52 in order to modify its length when modifying the hand support position, for example. Therefore, it can be adapted to the different positions of the hand support 6 to fit any type of hand. Advantageously, two of the binding means 51,51' comprise at least two preferably non-elastic tapes disposed so as to cross each other at the metacarpophalangeal joint of the patient's hand upon use. With this particular arrangement, the thumb and consequently also the hand of the patient is particularly well maintained in place, lowering the risk of having the hand releasing the compression sensing device. The other binding means 52 maintain the other fingers within the device "volume", i.e. between the protective shield and the hand support.

Figure 2 represents the same device in an exploded manner without the bindings to more clearly explain how it is assembled. By the way, it should be understood that the thumb support is actually placed inside the protective shield. The actual representation in figure 2 can be misinterpreted but plate 42 is under the thumb support 2 and is therefore assembled preferably before the thumb support to the handle 3.

A second aspect of the present invention relates to a compression sensing set comprising the compression sensing device according to the first aspect of the present invention, a processing module adapted to process the compression detected by the compression sensing device, and a display module adapted to display the signals and calculated results. Preferably, the compression sensing set further comprises an electrical stimulation device to generate several different stimulation profiles having a frequency lower than 10 Hz and/or larger than 30 Hz. Thanks to this, one can generate several types of electrical stimulation for an improved monitoring process.

A third aspect of the present invention relates to a compression sensing method using the compression sensing kit according to the second aspect of the present invention comprising the steps of detecting a compression force applied by the thumb on the force sensor through the thumb support, converting the detected compression into a standard force, converting said standard force into an electrical signal and sending it to the processing means, and displaying the detected force.

The present invention should not be limited to the above and it must be kept in mind that any modification to the above described invention that are made within the scope of the present invention as claimed in the appended claims are encompassed within the scope of the present patent application. For example, even if this is not represented here, the device can be made of any biocompatible material including transparent ones and the size of the device itself is not fixed and can be variable. Further, the shape of the several components of the device can be chosen according to any specific purpose of use. Finally, it must be understood that the protective shield protected here can be of any shape, any size, can even comprise openings or not, provided that it protects the thumb support from parasitic force that could induce artefacts during a monitoring process.

## Claims

1. Compression sensing device (1) for sensing an isometric thumb compression force applied by a thumb of a conscious or unconscious patient comprising
a handle (3) to be gripped by a hand of the patient,
a force sensor (31), and
a thumb support (2) mounted at a first end of the handle, for receiving a thumb of the hand of the patient in zero preload conditions and adapted to transmit compression force of the thumb to the force sensor,
**characterized in that** it further comprises a protective shield (4) attached to the handle and at least partially surrounding the thumb support so as to protect it from any external artefactual compression.

2. Compression sensing device according to claim 1, **characterized in that** it further comprises a hand support (6) movably mounted on a second end of the handle (3) opposed to the first end and being sloped with respect to said handle so as to be able to move along and around the handle's longitudinal direction.

3. Compression sensing device according to any one of claims 1 to 2, **characterized in that** the thumb support (2) is a bored component disposed so as to be rotatable around the longitudinal axis of the handle (3).

4. Compression sensing device according to claim 3, **characterized in that** the bored component (2) is a cylindrical element having a sloped support surface (21) adapted to receive the two phalanges of the thumb.

5. Compression sensing device according to any one of claims 1 to 4, **characterized in that** the zero preload positioning of the thumb on its support (2) permits to integrally transmit the isometric compression force of the thumb to the force sensor (31), which has a limited compression run.

6. Compression sensing device according to any one of claims 1 to 5, **characterized in that** the protective shield (4) comprises an opening (41) above the thumb support (2), adapted for receiving a calibration tool for calibration.

7. Compression sensing device according to any one of claims 1 to 6, **characterized in that** the protective shield (4) is detachably attached to the handle (3).

8. Compression sensing device according to any one of claims 1 to 7, **characterized in that** it further comprises binding means (51, 51', 52) adapted to maintain the hand in position on the compression sensing device (1).

9. Compression sensing device according to claim 8, **characterized in that** the binding means (51, 51', 52) are attached on the protective shield (4) on one end and on the hand support (6) on the other end.

10. Compression sensing device according to claim 9, **characterized in that** the binding means (51, 51', 52) comprise at least two non-elastic tapes (51, 51') disposed so as to cross each other at the metacarpophalangeal joint of the patient's hand upon use.

11. Compression sensing device according to claims 1 to 10, **characterized in that** the force sensor (31) is provided inside the handle (3).

12. Compression sensing kit comprising
the compression sensing device (1) according to any one of claims 1 to 11,
a processing module adapted to process the compression detected by the compression sensing device, and
a display module adapted to display the signals and the calculated results.

13. Compression sensing kit according to claim 12, **characterized in that** it further comprises an electrical stimulation generating means.

14. Compression sensing kit according to claim 13, **characterized in that** the electrical generating means are adapted to generate several different stimulation profiles having a frequency lower than 10 Hz and/or larger than 30 Hz.

15. Compression sensing method using the compression sensing kit of any one of claims 12 to 14 comprising the steps of
detecting a compression force applied by the thumb on the force sensor (31) through the thumb support (2),
converting the detected compression into a standard force converting said standard force into an electrical signal and sending it to the processing means
displaying the detected force.
